# EUROPEAN PATENT SPECIFICATION

(11) **EP 1 414 386 B1**
(45) Date of publication and mention of the grant of the patent: **28.09.2011**
(21) Application number: 02759332.6
(22) Date of filing: 09.08.2002
(51) Int. Cl.: A23L 1/30, A23K 1/16, A61K 31/232, A61K 31/195, A61K 31/355, A61K 31/375

(54) **CANINE CARDIAC DIET**
HERZDIÄT FÜR HUNDE
REGIME ALIMENTAIRE POUR CHIENS CARDIAQUES

(30) Priority: 10.08.2001 US 311547 P
(43) Date of publication of application: 06.05.2004
(73) Proprietor: Mars, Inc., McLean, VA 22101-3883 (US)
(72) Inventor: FREEMAN, Lisa, M., Westboro, MA 01581 (US); RUSH, John, E., Westboro, MA 01581 (US)
(74) Representative: Cornish, Kristina Victoria Joy
(86) International application number: PCT/US2002/025546
(87) International publication number: WO 2003/015695

(56) References cited:
- WO-A-91/11117
- WO-A-95/26646
- WO-A-98/47376
- WO-A-02/096221
- US-A- 5 895 652
- DATABASE WPI Section Ch, Week 199826 Derwent Publications Ltd., London, GB; Class B05, AN 1998-289843 XP002150137 & JP 10 099048 A (SNOW BRAND MILK PROD CO LTD) 21 April 1998 (1998-04-21)
- M.E. KEITH ET AL.: "Conditioned nutritional deficiencies in the cardiomyopathic hamster heart" CANADIAN JOURNAL OF CARDIOLOGY., vol. 17, no. 4, 2001, pages 449-458, XP008040492 XXPULSUS GROUP, INC.
- JOANNA DUKES MCEWAN: "Canine dilated cardiomyopathy 2. Pathophysiology and treatment" IN PRACTICE, vol. 22, no. 10, 2000, pages 620-626, XP008040487 GBBRITISH VETERINARY ASSOCIATION, LONDON
- DATABASE VETU [Online] (COLUMBUS, OHIO, USA) DUKES M.: 'Canine dilated cardiomyopathy 2. Pathophysiology and treatment', XP002959395 Retrieved from STN Database accession no. 2001-60247 & PRACT. vol. 22, no. 10, 2000, pages 620 - 626
- DATABASE MEDLINE [Online] (COLUMBUS, OHIO, USA) KEITH M. ET AL.: 'Conditioned nutritional deficiencies in the cardiomyopathic hamster heart', XP002959396 Retrieved from STN Database accession no. 2001325930 & CANADIAN J. CARDIOLOGY vol. 17, no. 4, April 2001, pages 449 - 458
- GOODWIN J ET AL: "The role of dietary modification and nondrug therapy in dogs and cats with congestive heart failure", VETERINARY MEDICINE, VETERINARY MEDICINE, EDWARDSVILLE, KS, US, vol. 93, no. 10, 1 October 1998 (1998-10-01), pages 919-926, XP009135674, ISSN: 8750-7943

## Description

The present invention provides a foodstuff comprising taurine, vitamin C, vitamin E and one or more n-3 polyunsaturated fatty acids, arginine and L-carnitine for use in the control of cardiovascular disorders, wherein said n-3 polyunsaturated fatty acids are provided at a level of from 50mg/400kcal or above, taurine is provided at a concentration of from 200mg to 1000mg/400kcal, carnitine is provided at a concentration of from 40mg to 400mg/400kcal and sodium is provided at 0.4g/400kcal or less

Cardiac disease is one of the most common disorders in dogs. Eleven percent of dogs in the United States and Europe have some form of cardiac disease. Of all dogs with cardiac disease, 95% have acquired disease while 5% have congenital disease. For dogs with acquired disease, the majority (75-80%) have endocardiosis or myxomatous degeneration of atrioventricular valves (commonly referred to as chronic valvular disease or CVD). Another 5-10% has dilated cardiomyopathy (DCM) and the remaining dogs with cardiac disease (10-20%) have pericardial disease, endocarditis, primary arrhythmias or heartworm disease.

Documents that address nutritional aspects of animals with cardiovascular problems are; Keith, M.E. et al (2001) can. J. Cardiology: 17, p449-458; Goodwin, J-K. et al (1998) Veterinary Medecine, 919-926; and Mc Ewan, J.D. (2000) In Practice, 22:620-626.

The present invention provides a foodstuff, which can optionally be used in combination with one or more conventional therapy to reduce the progression of cardiovascular disease. The foodstuff of the invention provides a further advantage as it utilises naturally occurring ingredients to provide a benefit to an animal which may in the future or present, suffer from a cardiovascular disease.

The first aspect of the invention provides a foodstuff comprising taurine, vitamin C, vitamin E and one or more n-3 polyunsaturated fatty acids, arginine and L-carnitine for use in controlling a cardiovascular disorder wherein said n-3 polyunsaturated fatty acids are provided at a level of from 50mg/400kcal or above, taurine is provided at a concentration of from 200mg to 1000mg/400kcal, carnitine is provided at a concentration of from 40mg to 400mg/400kcal and sodium is provided at 0.4g/400kcal or less.
The second aspect of the invention relates to the use of taurine, vitamin C, vitamin E and one or more n-3 polyunsaturated fatty acids, L-carnitine and arginine in the manufacture of a composition for the control of a cardiovascular disorder,

It is an object of the present invention, to provide a foodstuff, which improves the symptoms and/or slows the progression of a cardiovascular disease. It is beneficial since such a foodstuff can be administered to dogs with early, moderate and/or late stage cardiovascular disease. For the purposes of this invention, the term cardiovascular encompasses both disorders relating to or affecting the heart (cardiac) as well as the systemic and the pulmonary circulation (cardiovascular).

The foodstuff of the first aspect comprises taurine. Taurine is obtained from meat and fish and is a non-essential amino acid for some animals, such as dogs. It provides benefits to animals suffering from cardiovascular diseases, such as dilated cardiomyopathy. These benefits may be due to the positive inotropic effects of taurine or its role in calcium regulation in the myocardium.

For the purposes of the current invention, taurine is provided at a level from 200 mg to 1000 mg per 400kcal. Throughout this text, references to a concentration per kcal are to kcal metabolisable energy intake.

The foodstuff of the first aspect comprises vitamin C. Vitamin C is a water-soluble substance which has a number of important roles in the body. It has an essential role in the maintenance of healthy teeth, gums and bones. It aids the healing of wounds, scar tissue and fractures and strengthens blood vessels. Vitamin C also builds resistance to infection and aids in the prevention and treatment of the common cold. Vitamin C is also one of the major antioxidant nutrients.

Vitamin C for the present invention may be provided in any form, including as crystalline ascorbic acid (optionally pure), ethylcellulose coated ascorbic acid, calcium phosphate salts of ascorbic acid, ascorbyl palmitate, ascorbic acid-2-monophosphate salt or ascorbyl-2-monophosphate with small traces of the disphosphate salt and traces of the triphophate salt or calcium phosphate (Stay-C^{®}). Preferably, the vitamin C is provided as Stay-C^{®} or ascorbyl palmitate. The vitamin C according to the first aspect of the invention may be in any physical state. It may be liquid, semi-solid or solid (such as a powder).

Vitamin C for the purposes of this invention is provided at a level of from 4mg to 400mg per 400kcal per day, preferably from 20 mg to 200mg per 400kcal per day, more preferably at a level of from 30mg per 400kcal per day or above.

The foodstuff of the first aspect comprises vitamin E. Vitamin E is a collective term for several biologically similar compounds, including those called tocopherols and tocotrienols, which share the same biological activity. The selenium-containing enzyme glutathione peroxidase together with vitamin E helps to protect cells against free radical induced damage. Vitamin E acts as a scavenger of free radicals. Vitamin C may assist by reducing the tocopheroxyl radicals formed by the scavenging. In addition, vitamin E helps to block lipid peroxidation and may also form an important part of the membrane structure due to its interaction with membrane phospholipids. It has also been suggested that Vitamin E plays an important role in the functioning of the immune system. The most biologically active biological form of vitamin E in animal tissue is alpha-tocopherol. Vitamin E cannot be synthesised *in vivo.* Forms of vitamin E for the present invention are not limiting and include alpha-tocopherol, such as D-alpha-tocopherol, D-alpha-tocopherol acetate, DL-alpha-tocopherol and DL-alpha-tocopherol acetate.

Units of vitamin E can be expressed as International Units (IU), where 1 IU of alpha-tocopherol approximates to 1mg of alpha-tocopherol. Other vitamin E compounds have their IU determined by their biopotency in comparison to alpha-tocopherol as described in McDowell, L.R (1989) Vitamin E: In vitamins in Animal Nutrition, Chapter 4, page 96, Academic Press, UK.

Vitamin E is a major anti-oxidant nutrient and acts in the body as a free radical scavenger. Alpha-tocopherol is the most active anti-oxidant biological form of vitamin E.

The food supplement of the first aspect of the invention will preferably contain vitamin E at a level of from 1 IU to 1000 IU per 400kcal per day, preferably from 10 IU to 500 IU per 400kcal per day, more preferably at a level of 100 IU per 400kcal per day or above.

A further useful point in relation to the use of vitamin E in combination with vitamin C is their potential to act synergistically. This may be assisted by the fact that vitamin E is lipid soluble and vitamin C is water-soluble. Alpha-tocopherol is known to sit in the lipid membrane. Ascorbate and alpha-tocopherol, for example, interact at the interface between cell membranes or lipoproteins and water. Ascorbic acid rapidly reduces alpha-tocopherol radicals in membranes to regenerate alpha-tocopherol.

The foodstuff of the first aspect comprises one or more n-3 polyunsaturated fatty acid. Suitable polyunsaturated fatty acids include n-3 fatty acids (which include eicosapentaenoic acid (EPA), docasahexaenoic acid (DHA) and/or alpha-linolenic acid (ALA)).

Supplementation with n-3 polyunsaturated fatty acids provides a benefit to an animal by reducing the production of inflammatory eicosanoids (for example the 2-and 4-series eicosanoids) and therefore reducing inflammation. Futhermore, n-3 polyunsaturated fatty acids decrease the production of the inflammatory cytokines (including TNF and IL-1).

The form of any of the n-3 polyunsaturated fatty acid is not limiting and any one or more can be provided in a purified form or a non-purified form, such as: fish oil, fish meal, soya oil, blackcurrent oil, sunflower oil or ground nut oil. The fatty acids can further be obtained from flaxseed. Preferably, the fatty acid is provided by fish meal or fish oil.

The n-3 polyunsaturated fatty acid is provided at a level of from 50 mg per 400kcal or above.

The level of fatty acid provided in a foodstuff of the first aspect may vary depending on the particular fatty acid(s) and the type of food provided. The table below summarises preferred levels of eicosapentaenoic acid and docasahexaenoic acid for a wet or a dry foodstuff of the first aspect - all levels are provided per 400kcal.

| | **Wet foodstuff** | **Dry foodstuff** |
|---|---|---|
| **Level of Eicosapentaenoic acid** Preferred range | 50mg to 1000mg 200mg or above | 50mg to 500mg . 100mg or above |
| **Level of docasahexaenoic acid** Preferred range | 50mg to 1000mg 170mg or above | 50mg to 500mg 80mg or above |

The combination of the above ingredients has been shown to provide a benefit in terms of cardiovascular health of an animal.

The foodstuff of the first aspect of the invention may further comprise additional ingredients. Such additional ingredients, which may contribute to benefiting the cardiovascular health of an animal include one of more of a flavonoid, a vitamin, a mineral, arginine, magnesium and/or L-carnitine.

The intake of flavonoids in the diet results in a reduced risk of coronary heart disease. Flavonoids are anti-oxidant compounds which exhibit a number of useful properties for the control of cardiovascular disorders. A number of flavonoids have positive inotropic effects. In addition, flavonoids protect the heart against doxorubicin-induced cardiotoxicity, they have antiarrhythmic effects, they inhibit platelet aggregation and they inhibit nitric oxide production.

For the purposes of this invention, the form of any flavonoid is not limiting. It can be provided in an isolated and/or purified form or otherwise. In a preferred feature of the first aspect, one or more flavonoid is provided as a component of a raw material, for example fruit juice, skin, or pulp. Examples of a source of flavonoids include but are not limited to one or more of bilberry, tea, broccoli, blueberries, carrots, pomegranates, peanut skins, limes, soybeans, oranges, lemons, grapes, apples, tomatoes, onions or grapefruit.

Flavonoids are provided at a level of approximately 500mg to approximately 1 mg per 400kcal per day, preferably from approximately 100mg to approximately 10 mg per 400kcal per day. More preferably, one or more flavonoid are provided at a level of from 55 mg per 400kcal per day or above.

The foodstuff of the first aspect comprises L-carnitine. L-carnitine is concentrated in the skeletal and cardiac muscle. Carnitine deficiency is associated with primary myocardial disease in a number of dog species.

The foodstuff of the first aspect comprises L-carnitine at a level of from 40mg to 400mg per 400kcal.

The foodstuff of the first aspect optionally comprises a B complex vitamin. Low concentrations of the B complex vitamins and in particular thiamine have been implicated in congenstive heart failure. The foodstuff of the first aspect may therefore comprise a B complex vitamin at a level of from 0.01mg to 10mg per 400kcal per day, more preferably at a level of 0.24mg per 400 kcal or above. In particular thiamine may be provided at a level of from 1mg to 0.1mg per 400kcal, more preferably at a level of 0.23mg per 400kcal per day or above.

The foodstuff of the first aspect provides arginine, preferably L-arginine. The provision of L-arginine has been shown to improve endothelial dysfunction in animals with congestive heart failure. Furthermore, L-arginine supplementation has been shown to improve endothelium-dependent vasodilation and cardiac output. In addition, studies have shown that L-arginine reduced dyspnea in response to increasing carbon dioxide production during exercise in animals with severe chronic heart failure.

The present invention provides arginine at a level of from 0.5g to 10g per 400kcal per day, preferably from 1g to 5g per 400kcal per day. More preferably, arginine is provided at a level of from 2.4g per 400kcal per day or above.

The foodstuff of the first aspect may optionally provide magnesium. Magnesium is an essential prosthetic group in many enzymatic reactions involving carbohydrate and fatty acid metabolism, protein and nucleic acid synthesis, the adenylate cyclase system and cardiac and smooth muscle contractility. Thus, magnesium plays an important role in normal cardiac function. It is also clear that alterations in magnesium hemeostasis can have deleterious effects in a variety of cardiovascular conditions including hypertension, coronary disease, congestive heart failure, and cardiac arrhythmias. In addition, numerous drugs used to treat cardiac conditions, including digoxin and loop diuretics are associated with magnesium depletion. Therefore, animals with heart failure receiving these medications have the potential to develop hypomagnesemia. Hypomagesemia can increase the risk of arrhythmias, decrease cardiac contractility, and can potentiate the adverse effects of cardiac medications.

The foodstuff of the first aspect of the present invention therefore, provides magnesium at a level of from 0.01g to optionally 1g per 400 kcal per day, preferably 0.03g to 0.5g per 400 kcal per day. More preferably, magnesium is provided at a level of 0.08g per 400 kcal per day or above.

the first aspect, the foodstuff contains restricted levels of sodium. Sodium restriction is one method, together with the use of diuretics and venous vasodilators, to treat excessive increases in preload in animals with congenital heart failure.

Studies using low sodium diets in dogs with heart failure secondary to either endocardiosis or dilated cardiomyopathy have shown that serum sodium and chloride decreased significantly in dogs while eating a low sodium diet compared to a moderate sodium diet. Neurohormones did not change significantly on the low-sodium diet compared to the moderate-sodium diet, especially in dogs with endocardiosis.

This suggests that there may be benefits to a low-sodium diet in dogs with Class II-IV heart failure. Thus a foodstuff for an animal with no symptoms of cardiovascular disease or early stage cardiovascular disease may have no restrictions on the level of sodium. The foodstuff contains 0.4g of sodium per 400kcal or less.

A foodstuff for an animal with late stage cardiovascular disease will preferably have a restricted sodium level, for example from 0.2g of sodium per 400kcal per day or less.

The present invention relates to dogs. The dog includes the domestic dog (*Canis domesticus*).

The foodstuff of the invention may be a dry product (with approximately 5 to 12% moisture), a semi-moist product (with approximately 12 to 70% moisture) or a wet product (with approximately 70 to 90% moisture).

The foodstuff of the first aspect may further be provided to an animal as a mixture of wet and dry food. Such a combination may be provided premixed or may be provided as two or more separate foodstuffs, which are administered to the animal separately, simultaneously or sequentially. As previously discussed, the foodstuff of the first aspect can be provided as a food supplement which is added to an animal's conventional foodstuff.

The foodstuff according to the present invention encompasses any product that an animal consumes in its diet. In particular, the product is a pet food for a pet animal, more particularly a dog food for a dog. Thus, the invention covers standard food products as well as pet food snacks (for example, snack bars, biscuits and sweet products). The foodstuff is preferably a cooked product. It may incorporate meat or animal derived material (such as beef, chicken, turkey, lamb, fish, blood plasma, marrow bone etc or one or more thereof). The product alternatively may be meat free (preferably including a meat substitute such as soya, maize gluten or a soya product) in order to provide a protein source. The product may contain additional protein sources such as soya protein concentrate, milk proteins, gluten etc.

The product may also contain a starch source such as one or more grains (e.g. corn, rice, oats, barley etc), or may be starch free. It may include a gelatinised starch matrix.

The foodstuff is preferably packaged. In this way, the consumer is able to identify, from the packaging, the ingredients in the foodstuff and confirm that it is suitable for the particular pet in question. The packaging may be metal (usually in the form of a tin or flexifoil), plastic (usually in the form of a pouch or bottle), paper or card. The amount of moisture in any product may influence the type of packaging, which can be used or is required.

The foodstuff of the invention is preferably a complete and balanced food or is preferably used in combination with a complete and balanced food (for example, as described in National Research Council, 1985, Nutritional Requirements for Dogs, National Academy Press, Washington D.C. or Association of American Feed Control Officials, Official Publication 1996). A complete and balanced diet includes a high quality commercial food. A high quality commercial food can be defined as a diet manufactured to the nutrient recommendations of the National Research Council, 1985 (*supra*), wherein the digestibility of key nutrients is 80% or more.

The concentrations of the components to be added to the foodstuff are calculated on the basis of the energy content of the foodstuff and of any additional nutrients which may be consumed by the animal. Preferably, a complete and balanced food, (including a high quality commercial food) comprises the foodstuff according to the invention.

The foodstuff of the first aspect can be provided as a food supplement. The food supplement can be a powder, biscuit, kibble, sauce, topping, pocket or tablet that can be administered with or without an additional foodstuff. Where the food supplement is administered with an additional foodstuff, the food supplement can be administered sequentially simultaneously or separately. The food supplement may be mixed with the foodstuff, sprinkled or poured over the foodstuff or served separately. Alternatively, the food supplement can be added to a liquid provided for drinking such as water or milk.

One problem associated with animals with cardiovascular disorders is that they do not ingest sufficient calories. This is a particular problem in animals with advanced stage cardiovascular disorders, when the animal may have anorexia and/or weight loss. The first aspect of the invention therefore provides a foodstuff in the form of a calorie dense snack or treat. The foodstuff is preferably in the form of a bar. This snack or treat is provided as a nutritional supplement. The snack or treat is preferably highly palatable, more preferably having a sweet flavour. The snack or treat is calorie dense providing 100 to 500kcal per 65g bar more preferably 200 to 300kcal per 65g bar, more preferably 240kcal per 65g bar or above.

The foodstuff is for use in controlling a cardiovascular disorder.

For the purposes of this invention, cardiovascular disorders comprise acquired and congenital cardiovascular disorders. Such disorders include one or more of chronic valvular disease, dilated cardiomyopathy, congestive heart failure and hypertension.

Some animals with cardiovascular disorders do not exhibit clinical signs of the disorder. Other animals exhibit objective evidence of a disorder including cardiac murmurs, arrhythmia, and gallop on auscultation. The foodstuff of the first aspect may be administered to animals with cardiovascular disorders wherein the animals display clinical symptoms of the disorder. Alternatively, the foodstuff may be administered to animals susceptible to one or more cardiovascular disorders. Animals with cardiovascular disorders can go on to develop congestive heart failure in which inadequate cardiac performance results in excessive fluid retention. This excess fluid retention can result in conditions including pulmonary oedema, pleural effusion, ascites and pericardial effusion. The invention therefore relates to a foodstuff for use in controlling congestive heart failure.

The invention further relates to controlling a pericardial disorder including pericardial endocarditis, primary arrhythmias and heartworm disease.

Dogs with cardiac disease can be classified according to their clinical signs based on the modified New York Heart Association (NYHA) classification, as set out below.
Class I : Dogs have no limitations. Physical activity, including normal exercise, does not cause symptoms.
Class II : Slight limitation of physical activity and ordinary physical activity results in symptoms.
Class III : Marked limitation of physical activity so that less than ordinary activity leads to symptoms.
Class IV : Inability to carry out any activity without symptoms. Symptoms present at rest.

For the purposes of this invention, dogs classified as class I and II have early cardiovascular disease and classes III and IV have late stage cardiovascular disease.

The foodstuff of the first aspect can be administered to an animal with early stage or late stage cardiovascular disease. It will be appreciated that the nutritional requirements of an animal with late stage cardiovascular disease may differ from an animal with early stage cardiovascular disease. Therefore, in a preferred feature of the first aspect, a foodstuff for administering to an animal with early stage cardiovascular disease may have a different composition to a foodstuff for administering to an animal with late stage cardiovascular disease. For example, a foodstuff for administering to an animal with late stage cardiovascular disease may comprise restricted levels of sodium or may provide more calories than a foodstuff for administering to an animal with early stage cardiovascular disease.

The foodstuff of the first aspect can be administered to animals susceptible to a cardiovascular disorders. Such animals, while not presently suffering from a cardiovascular disorder may be prone to developing such a disorder in the future. Administering a foodstuff of the first aspect to a susceptible animal may delay the onset of the cardiovascular disorder or may reduce the severity of the symptoms thereof. Animals may be susceptible to one or more cardiovascular disorders for a variety of reasons including age, environmental conditions, family history, diet, genetic factors etc. Certain breeds of dogs for example, large and giant breeds, are susceptible to cardiovascular disorders. Such dogs include but are not limited to Doberman Pinschers, Boxers, Great Danes, Scottish Deerhounds, Irish Wolfhounds and Cocker Spaniels. Dogs which are susceptible to valvular disease include small breeds such as, but not limited to, Cavalier King Charles Spaniels.

In addition, the foodstuff of the first aspect can be used to aid in the control of secondary symptoms such as anorexia, cachexia, weight loss, fluid retention (e.g. manifested as pulmonary oedema, pleural effusion, ascites, pericardial effusion), exercise intolerance, coughing and oxidative stress which occur with one or more of the cardiovascular disorder discussed above.

For the purposes of this invention, the terms "control" and "controlling" mean to decrease or alleviate the symptoms suffered by an animal especially the symptoms of a cardiovascular disorder and/or assist in the management of a cardiovascular disorder. This foodstuff may be provided as an adjunct therapy and is preferably provided in combination with a conventional treatment.

Such conventional treatment may include diurectics such as flurosemide, digoxin or spironolactone, or Angiotensin Converting Enzyme (ACE) pm inhibitors such as captorpril or enalopril.

The foodstuff of the invention may allow the reliance on a conventional treatment such as diurectics or Angiotensin Converting Enzyme (ACE) inhibitors to be reduced. Alternatively the animal may exhibit less symptoms or the severity of the symptoms may be reduced. The animal may exhibit an improved level of well being, for example, exemplified by an increase in activity.

It is intended that the foodstuff of the first aspect will be provided as a commercial product, which will be available from commercial outlets and/or from veterinary surgeons. The foodstuff of the first aspect may be provided as required, under the direction of a veterinary surgeon. The foodstuff may be fed in combination with one or more specific treatments for a cardiac disorder under the guidance of a veterinary surgeon. The foodstuff may be branded as a dietary aid, or complete foodstuff.

The second aspect of the invention relates to the use of taurine, vitamin C, vitamin E and one or more n-3 polyunsaturated fatty acids, L-carnitine and arginine in the manufacture of a composition for the control of a cardiovascular disorder, wherein said n-3 polyunsaturated fatty acids are provided at a level of from 50mg/400kcal or above, taurine is provided at a concentration of from 200mg to 1000mg/400kcal, carnitine is provided at a concentration of from 40mg to 400mg/400kcal and sodium is provided at 0.4g/400kcal or less.

All preferred features of the first aspect, also apply to the second aspect.

The foodstuff is administered one or more times per day. The foodstuff can be administered in combination with or in place of the animal's conventional food. Where the foodstuff of the first aspect is a calorie dense snack or treat, the foodstuff can be administered in addition to the animal's conventional food. The calorie dense snack or treat can be administered one or more times daily, or can be administered as required (for example, if the animal is undergoing physical exercise, work or training or if the animal requires an increase in its calorific intake).

The preferred form of the foodstuff is a pet food product. The process for making the pet food product comprises mixing together the ingredients for the pet food product and incorporating the components vitamin C, vitamin E, taurine, L-carnitine and arginine and one or more n-3 polyunsaturated fatty acid. The components may be added at any time during the manufacturing/processing of the foodstuff including as the last step before packaging.

The foodstuff can be made according to any method known in the art such as in Waltham Book of Dog and Cat Nutrition, Ed. ATB Edney, Chapter by A. Rainbird, entitled "A Balanced Diet" in pages 57 to 74 Pergamon Press Oxford.

The components are added together at any time during the processing. They may all be added together at the same time, or individually, in any particular order. Other ingredients of the foodstuff may be added at any time during the processing.

Where the foodstuff is a dry product, preferably, two or more ingredients of the foodstuff are mixed together and then ground together. The moisture and temperature of the ground particles can be manipulated prior to any further processing step. The components may be added before or after any heating or cooking step. The processing may include shaping and/or packaging of the product. In a preferred feature of the fifth aspect, the product is shaped by extrusion to form pellets or kibbles. Extrusion preferably occurs at a pressure of 20-1000 psig and a temperature of 90-165°C.

The essential components of the foodstuff (taurine, vitamin C, vitamin E and one or more polyunsaturated fatty acid) may be mixed with the other components of the foodstuff or can be added to the completed foodstuff. In a preferred feature of the invention, one or more of the essential components is coated or sprayed on to the surface of the foodstuff. Alternatively, one or more of the essential components is present as part of a soft centre. Alternatively, one or more of the essential components is admixed with one or more other components of the foodstuff. The final water content of the foodstuff can be manipulated using a cooler apparatus.

Where the foodstuff is a wet product, preferably two or more ingredients of the foodstuff are mixed together and then optionally cooked or heated. The essential components of the foodstuff may be added before or after any heating or cooking step. The processing may include shaping and/or packaging of the product.

### Examples

### Example 1

### Example of cardiovascular diet not according to the invention

The following cardiovascular diet is a dry diet containing approximately 10% water. The dry diet will provide approximately 300kcal per 100g or above. The dry cardiovascular diet for animals with early stage disease comprises the following ingredients (~ is approximately):

Percentage is calculated on an "as is" weight for weight basis

| | |
|---|---|
| Rice | ~58% |
| Fish meal | ~10% |
| Poultry | ~10% |
| Rice gluten | ~12.5 |
| Fibre | ~4% |
| Vitamins and minerals | ~5% |
| (including Vitamin C | ~0.03%) |
| Taurine | ~0.1% |
| Carnitine | ~0.05% |
| Antioxidant complex | ~0.25% |

The above cardiovascular diet provides from 20g to 28g of protein per 400kcal per day. Sodium is provided at a level of approximately 0.01 to 0.6g per 400 kcal per day.

### Example 2

### Example of late stage cardiovascular diet, not according to the invention

The following diet is a dry diet with a composition as set out in Example 1. It will be appreciated that the foodstuff for administration to an animal with advanced stage disorder contains a lower level of sodium, for example from approximately 0.01g per 400kcal per day or above.

The late stage cardiovascular diet provides from 20g to 28g of protein per 400kcal per day.

### Example 3

### Manufacture of dry food

Dry raw materials are weighed, mixed and ground. The dry mix is then screened prior to extrusion to form the mixed meal. The mixed meal is conveyed to a pre-conditioner where it is mixed with steam, water and oil at specified rates. Sufficient residence time is provided in the pre-conditioner for the moisture and temperature to transfer uniformly throughout the individual particles. The residence time is about two minutes. The pre-conditioned mixture is then transferred to an extruder for cooking and forming. The die pressure should be 200 - 1000 psig and the die temperature is about 90 - 165 °C. The formed kibble is pneumatically conveyed to the dryer. The drying temperature is set at 130 - 145 °C and drying time is about 17 minutes. The product moisture exiting the dryer is less than 12%. The dried kibble is sized (or screened) before coating to reduce clumps and fines. The kibble is fed into a coating system where coating is applied uniformly across the surface of the kibble at a constant application rate. The coating materials include digest The kibble is coated with digest at ambient temperature. The coated kibble is then transferred to a post-coat cooler in which the coated product is conditioned to its final moisture (< 12%), water activity (<0.7 at 35 °C) and temperature (< 35 °C) prior to packaging. The retention time is about 15 minutes.

### Example 4

### Effect of foodstuff not according to the invention on dogs.

A panel of healthy dogs underwent a feeding trial to compare the effects of feeding the foodstuff of the present invention with a control diet. The control diet contained the same ingredients as the foodstuff minus vitamin C, vitamin E, taurine and one or more polyunsaturated fatty acid. The dogs fed with the foodstuff showed a benefit compared to those on the control diet. Further, no deleterious side effects were observed with the foodstuff.

### Example 5

### Supplement bar

In addition to the essential ingredients, a formed supplement bar provides approximately 250kcal per 65g bar and contains from approximately 0.04 to approximately 0.06g of sodium per 100kcal. Protein is provided by the bar at a level of approximately 4 to approximately 5g per 100kcal.

## Claims

1. A foodstuff for a dog comprising vitamin C, taurine, vitamin E, one or more n-3 polyunsaturated fatty acids, arginine, L-carnitine, for use in controlling a cardiovascular disorder, wherein said n-3 polyunsaturated fatty acids are provided at a level of from 50mg / 400kcal or above, the taurine is provided at a concentration of from 200 mg to 1000mg per 400kcal, carnitine is provided at a concentration of from 40mg to 400mg per 400kcal, and sodium is provided at 0.4g/400kcal or less

2. A foodstuff as claimed in claim 1 wherein the n-3 polyunsaturated fatty acid is selected from one or more of eicosapentaenoic acid, docasahexaenoic acid or alpha-linolenic acid.

3. A foodstuff as claimed in any one of claims 1 to 2 further comprising one or more B complex vitamins.

4. A foodstuff as claimed in any one of claims I to 3 further comprising one or more flavonoids.

5. A foodstuff as claimed in any one of claims 1 to 4 further comprising magnesium.

6. A foodstuff as claimed in any one of claims 1 to 6 wherein vitamin C is provided at a concentration of from 4mg to 400mg per 400kcal.
sodium is provided at 0.4g/400kcal or less

7. A foodstuff as claimed in any one of claims 1 to 8 wherein vitamin E is provided at a concentration of from 11 IU to 1000 IU per 400kcal.

8. A foodstuff as claimed in any one of claims 1 to 7 wherein the cardiovascular disorder is acquired or congenital.

9. A foodstuff as claimed in claim 8 wherein the acquired cardiovascular disorder is one or more of chronic valvular disease, dilated cardiomyopathy, congestive heart failure or hypertension.

10. A foodstuff as claimed in claim 8 wherein the acquired cardiovascular disorder is one or more of a pericardial disease, endocarditis, primary arrhythmia or heartworm disease.

11. The use of vitamin C, taurine, vitamin E, one or more n-3 polyunsaturated fatty acids arginine and L-carnitine in the manufacture of a composition for the control of a cardiovascular disorder, wherein said n-3 polyunsaturated fatty acids are at a level of from 50mg/400kcal or above, taurine is at a concentration of from 200mg to 1000mg/400kcal, carnitine is provided at a concentration of from 40mg to 400mg per 400kcal and sodium is provided at 0.4g/400kcal or less.

12. A use as claimed in claim 11 wherein the cardiovascular disorder is acquired or congenital.

13. The use as claimed in claim 12 wherein the acquired cardiovascular disorder is one or more of chronic valvular disease, dilated cardiomyopathy, congestive heart failure or hypertension.

14. The use as claimed in claim 12 wherein the acquired cardivascular disorder is one or more of a pericardial disease, endocarditis, primary arrhythmia or heartworm disease.

## Patentansprüche

1. Futtermittel für einen Hund, umfassend Vitamin C, Taurin, Vitamin E, eine oder mehrere n-3 mehrfach ungesättigte Fettsäuren, Arginin und L-Camitin, zur Verwendung bei der Kontrolle einer kardiovaskulären Erkrankung, wobei die n-3 mehrfach ungesättigten Fettsäuren mit einem Gehalt von 50 mg/400 kcal oder mehr bereitgestellt sind, das Taurin mit einer Konzentration von 200 mg bis 1000 mg pro 400 kcal bereitgestellt ist, Carnitin mit einer Konzentration von 40 mg bis 400 mg pro 400 kcal bereitgestellt ist und Natrium mit 0,4 g/400 kcal oder weniger bereitgestellt ist.

2. Futtermittel nach Anspruch 1, wobei die n-3 mehrfach ungesättigte Fettsäure ausgewählt ist aus einer oder mehreren von Eicosapentaensäure, Docosahexaensäure oder Alpha-Linolensäure.

3. Futtermittel nach einem der Ansprüche 1 bis 2, das weiter ein oder mehrere B-Komplex-Vitamine umfasst.

4. Futtermittel nach einem der Ansprüche 1 bis 3, das weiter ein oder mehrere Flavonoide umfasst.

5. Futtermittel nach einem der Ansprüche 1 bis 4, das weiter Magnesium umfasst.

6. Futtermittel nach einem der Ansprüche 1 bis 5, wobei Vitamin C mit einer Konzentration von 4 mg bis 400 mg pro 400 kcal bereitgestellt ist.

7. Futtermittel nach einem der Ansprüche 1 bis 6, wobei Vitamin E mit einer Konzentration von 11 IU bis 1000 IU pro 400 kcal bereitgestellt ist.

8. Futtermittel nach einem der Ansprüche 1 bis 7, wobei die kardiovaskuläre Erkrankung erworben oder angeboren ist.

9. Futtermittel nach Anspruch 8, wobei die erworbene kardiovaskuläre Erkrankung eine oder mehrere von chronischer Herzklappenerkrankung, dilatierter Kardiomyopathie, kongestivem Herzversagen oder Bluthochdruck ist.

10. Futtermittel nach Anspruch 8, wobei die erworbene kardiovaskuläre Erkrankung eine oder mehrere von einer perikardialen Erkrankung, Endokarditis, primärer Herzrhythmusstörung oder Divofilaria-immitis-Erkrankung ist.

11. Verwendung von Vitamin C, Taurin, Vitamin E, einer oder mehreren n-3 mehrfach ungesättigten Fettsäuren, Arginin und L-Carnitin zur Herstellung einer Zusammensetzung zur Kontrolle einer kardiovaskulären Erkrankung, wobei die n-3 mehrfach ungesättigten Fettsäuren mit einem Gehalt von 50 mg/400 kcal oder mehr bereitgestellt sind, Taurin mit einer Konzentration von 200 mg bis 1000 mg/400 kcal bereitgestellt ist, Carnitin mit einer Konzentration von 40 mg bis 400 mg pro 400 kcal bereitgestellt ist und Natrium mit 0,4 g/400 kcal oder weniger bereitgestellt ist.

12. Verwendung nach Anspruch 11, wobei die kardiovaskuläre Erkrankung erworben oder angeboren ist.

13. Verwendung nach Anspruch 12, wobei die erworbene kardiovaskuläre Erkrankung eine oder mehrere von chronischer Herzklappenerkrankung, dilatierter Kardiomyopathie, kongestivem Herzversagen oder Bluthochdruck ist.

14. Verwendung nach Anspruch 12, wobei die erworbene kardiovaskuläre Erkrankung eine oder mehrere von einer perikardialen Erkrankung, Endokarditis, primärer Herzrhythmusstörung oder Divofilaria-immitis-Erkrankung ist.

## Revendications

1. Un aliment pour un chien comprenant de la vitamine C, de la taurine, de la vitamine E, un ou plusieurs acides gras 1-3 polyinsaturés, de l'arginine et de la L-carnitine, destiné à être employé dans le contrôle de troubles cardiovasculaires, dans lequel lesdits acides gras 1-3 polyinsaturés sont présents à un taux de 50mg/400kcal ou plus, la taurine est présente en une concentration allant de 200mg à 1000mg par 400kcal, la carnitine est présente en une concentration allant de 40mg à 400mg par 400kcal et le sodium est présent en une concentration de 0,4g/400kcal ou inférieure.

2. Un aliment tel que revendiqué dans la revendication 1, dans lequel 1' acide gras 1-3 polyinsaturé est sélectionné parmi un ou plusieurs acide eicosapentaenoïque, acide docasahexaenoïque et acide alpha-linolénique.

3. Un aliment tel que revendiqué dans l'une quelconque des revendications 1 ou 2, comprenant en outre une ou plusieurs vitamines B complexées.

4. Un aliment tel que revendiqué dans l'une quelconque des revendications 1 à 3, comprenant en outre un ou plusieurs flavonoïdes.

5. Un aliment tel que revendiqué dans l'une quelconque des revendications 1 à 4, comprenant en outre du magnésium.

6. Un aliment tel que revendiqué dans l'une quelconque des revendications 1 à 6, dans lequel la vitamine C est présente en une concentration allant de 4mg à 400mg par 400kcal.

7. Un aliment tel que revendiqué dans l'une quelconque des revendications 1 à 8, dans lequel la vitamine E est présente en une concentration allant de 11 IU à 1000 IU par 400kcal.

8. Un aliment tel que revendiqué dans l'une quelconque des revendications 1 à 7, dans lequel le trouble cardiovasculaire est un trouble acquis ou congénital.

9. Un aliment tel que revendiqué dans la revendication 8, dans lequel le trouble cardiovasculaire acquis est un ou plusieurs des troubles de la liste suivante: valvulopathie chronique, myocardiopathie dilatée, insuffisance cardiaque congestive ou hypertension.

10. Un aliment tel que revendiqué dans la revendication 8, dans lequel le trouble cardiovasculaire acquis est un ou plusieurs des troubles de la liste suivante: maladie péricardique, endocardite, arythmie cardiaque primaire, maladie du ver du coeur.

11. L'utilisation de vitamine C, de taurine, de vitamine E, de un ou plusieurs acides gras 1-3 polyinsaturés, d'arginine et de L-carnitine, pour la fabrication d'une composition destinée à être employée pour le contrôle de trouble cardiovasculaire, dans laquelle lesdits acides gras 1-3 polyinsaturés sont présents à un taux de 50mg/400kcal ou plus, la taurine est présente en une concentration allant de 200mg à 1000mg par 400kcal. la carnitine est présente en une concentration allant de 40mg à 400mg par 400kcal et le sodium est présent en une concentration de 0,4g/400kcal ou inférieure.

12. Une utilisation telle que revendiquée dans la revendication 11, dans laquelle le trouble cardiovasculaire est un trouble acquis ou congénital.

13. Une utilisation telle que revendiquée dans la revendication 12, dans laquelle le trouble cardiovasculaire acquis est un ou plusieurs des troubles de la liste suivante: valvulopathie chronique, myocardiopathie dilatée, insuffisance cardiaque congestive ou hypertension.

14. Une utilisation telle que revendiquée dans la revendication 12, dans laquelle le trouble cardiovasculaire acquis est un ou plusieurs des troubles de la liste suivante: maladie péricardique, endocardite, arythmie cardiaque primaire, maladie du ver du coeur.
